**Europäisches Patentamt**

**European Patent Office**

**Office européen des brevets**

(19)

(11) Veröffentlichungsnummer : **0 148 319
B1**

# (12) EUROPÄISCHE PATENTSCHRIFT

(45) Veröffentlichungstag der Patentschrift :
**13.04.88**

(21) Anmeldenummer : **84109819.7**

(22) Anmeldetag : **17.08.84**

(51) Int. Cl.⁴ : **A 61 M   1/30**, A 61 M   1/14

(54) **Dialysevorrichtung.**

(30) Priorität : **01.10.83 DE 3335744**

(43) Veröffentlichungstag der Anmeldung :
**17.07.85 Patentblatt 85/29**

(45) Bekanntmachung des Hinweises auf die Patenterteilung : **13.04.88 Patentblatt 88/15**

(84) Benannte Vertragsstaaten :
**FR GB IT SE**

(56) Entgegenhaltungen :
**EP-A- 0 071 951
DE-A- 2 441 210
DE-B- 2 236 433
US-A- 3 830 234**

(73) Patentinhaber : **INTERMEDICAT GMBH
Gerliswilstrasse 74
CH-6020 Emmenbrücke (CH)**

(72) Erfinder : **Rath, Dieter
Franz-Gleim-Strasse 69
D-3508 Melsungen (DE)**
Erfinder : **Seeck, Peter
Obermelsunger Strasse 32
D-3508 Melsungen (DE)**

(74) Vertreter : **von Kreisler, Alek, Dipl.-Chem. et al
Deichmannhaus am Hauptbahnhof
D-5000 Köln 1 (DE)**

**Beschreibung**

Die Erfindung betrifft eine Dialysevorrichtung zum Dialysieren des Blutes eines Patienten, mit einer einzigen einlumigen Kanüle zum Punktieren eines Blutgefäßes, einer von der Kanüle abgehenden Blutleitung, die sich in eine Arterienleitung und eine Venenleitung verzweigt, zwei abwechselnd betätigten Absperrventilen in der Arterienleitung und der Venenleitung, einer über das erste Absperrventil an die Arterienleitung angeschlossenen einzigen Pumpe, einem zwischen Pumpe und Venenleitung geschalteten Dialysator und mit einer Expansionskammer zur vorübergehenden Aufnahme von Blut.

Es sind verschiedene Dialysevorrichtungen für die Einnadeldialyse bekannt. Bei der Einnadeldialyse wird eine einzige einlumige Kanüle in ein Blutgefäß des Patienten eingestochen. An die Kanüle ist eine Blutleitung angeschlossen, die sich in eine Arterienleitung und eine Venenleitung verzweigt. Eine Blutpumpe fördert das Blut von dem Patienten über die Arterienleitung in eine Expansionskammer. In einer zweiten Phase wird die Arterienleitung abgesperrt und die Venenleitung geöffnet. Das Blut wird nun aus der Expansionskammer zu einem Dialysator gepumpt und von dort über die geöffnete Venenleitung dem Patienten wieder zugeführt. In der Regel werden zwei Pumpen eingesetzt, von denen die eine Blut aus der Arterienleitung ansaugt und die andere Blut in die Venenleitung hineinpumpt. Als Pumpen werden Schlauchpumpen eingesetzt, deren Fördermenge der Pumpendrehzahl proportional ist.

Bei einer bekannten Dialysevorrichtung der eingangs genannten Art (EP-A-71 951) ist eine elastisch aufweitbare Expansionskammer zur vorübergehenden Aufnahme des durch die Arterienleitung angesaugten Blutes zwischen der Pumpe und dem Dialysator angeordnet. Der Expansionskammerauslaß führt zu dem Dialysator. Der Dialysatorauslaß ist mit der Venenleitung verbunden. Die Arterienleitung und die Venenleitung enthalten je ein Absperrventil, wobei beide Absperrventile abwechselnd betätigt werden, so daß jeweils eine der beiden Leitungen verschlossen und die andere geöffnet ist. In der Einlaufphase ist das Absperrventil der Arterienleitung geöffnet. Die Pumpe saugt dann über die Arterienleitung Blut in die Expansionskammer ein. Wenn das Volumen der Expansionskammer auf einem bestimmten Wert angestiegen ist, wird das Absperrventil in der Arterienleitung geschlossen und das Absperrventil in der Venenleitung geöffnet. Das in der Expansionskammer befindliche Blut wird durch die weiterlaufende Blutpumpe und die elastische Expansionskammerwandung aus der Expansionskammer abgeführt und über den Dialysator der Venenleitung zugeführt. Die Umschaltung zwischen der Einlaufphase und der Rücklaufphase erfolgt jeweils volumenabhängig. Die Volumenmessung erfordert eine aufwendige elastische Expansionskammer, deren Elastizitätseigenschaften sich im Laufe der Zeit verändern können, so daß die Umschaltpunkte nicht mit hinreichender Genauigkeit eingehalten werden. Außerdem besteht die Möglichkeit, daß die Grenzwerte der Umschaltung fehlerhaft eingestellt werden.

Ein weiterer Nachteil der bekannten Dialysevorrichtung besteht darin, daß die permanent laufende Pumpe ständig in die Venenleitung pumpt. Wenn die Venenleitung abgesperrt ist, erhöht sich der Druck in ihr, bis das Grenzvolumen der Expansionskammer erreicht ist. Ist der Grenzwert erreicht, dann wird das Absperrventil in der Venenleitung geöffnet und der Druck entlädt sich über die Kanüle in das Blutgefäß hinein. Gleichzeitig läuft aber die Pumpe immer noch weiter, so daß sich die normale Pumpenfördermenge der Druckentladung noch überlagert. Hierdurch entstehen Schwankungen der Blutfördermenge, die dem Patienten zugeführt wird.

Der Erfindung liegt die Aufgabe zugrunde, eine Dialysevorrichtung der eingangs genannten Art zu schaffen, die in der Einlaufphase das Blut mit konstanter Förderrate aus dem Patienten abzieht und in der Rücklaufphase das Blut dem Patienten mit ebenfalls konstanter Förderrate, also ohne Druckentladung, wieder zuführt und bei der gleichzeitig der Abbau des Druckes in der Expansionskammer über die Blutpumpe erfolgt.

Zur Lösung dieser Aufgabe ist erfindungsgemäß vorgesehen, daß die Expansionskammer über je ein Absperrventil mit dem Auslaß und mit dem Einlaß der Pumpe verbunden ist.

Nach der Erfindung wird die Blutmenge, die dem Patienten entzogen wird, in der Einlaufphase ausschließlich durch die Fördergeschwindigkeit der Pumpe bestimmt. Die Pumpe fördert aus der Arterienleitung in die Expansionskammer hinein. Es kann sich kein Druck in Richtung auf die Venenleitung ausbilden. Während der Rücklaufphase fördert die Pumpe das Blut aus der Expansionskammer über den Dialysator in die Venenleitung. Hierbei kann sich der Druck, der sich in der Expansionskammer aufgebaut hat, nicht schlagartig entladen, da der Abbau dieses Druckes über die Blutpumpe erfolgt.

Die erfindungsgemäße Dialysevorrichtung arbeitet mit einer einzigen einlumigen Kanüle und mit einer einzigen Pumpe. Diese Pumpe wird sowohl zum dosierten Auffüllen der Expansionskammer als auch zum dosierten Entleeren der Expansionskammer benutzt. In der Einlaufphase gelangt das Blut über die Pumpe in die Expansionskammer und in der Rücklaufphase verläßt das Blut die Expansionskammer wiederum über die Pumpe, um dem Dialysator zugeführt zu werden. Die Blutmenge, die der Arterienleitung entnommen wird, hängt ausschließlich von der Pumpendrehzahl ab und diejenige Blutmenge, die in die Venenleitung hineingepumpt wird, hängt ebenfalls ausschließlich von der Pumpendrehzahl ab. Auf diese Weise wird das Blutsystem des Patienten keinen großen zeitlichen Druckschwankungen ausgesetzt.

Die Merkmale des Anspruchs 7 ermöglichen eine sehr genaue Steuerung der Zeitabläufe von Einlaufphase und Rücklaufphase, ohne für den Steuerungsablauf Druckmessungen durchführen zu müssen. Andererseits kann die Förderrate der Pumpe entweder von Hand verändert werden oder programmgemäß unterschiedliche Werte zwischen Einlaufphase und Rücklaufphase annehmen. Da die Fördermenge der Pumpe der Pumpendrehzahl proportional ist, kann auf einfache Weise die Zeit errechnet werden, die die Pumpe benötigt, um die Expansionskammer mit Blut zu füllen bzw. zu entleeren. Die Fördergeschwindigkeit der Pumpe kann während jeder Phase verändert werden. In jedem Fall ist sichergestellt, daß das Füllvolumen der Expansionskammer in der Einlaufphase erreicht und in der Rücklaufphase abgepumpt wird. Druckmessungen können an verschiedenen Stellen des Systems zur Funktionsüberwachung vorgesehen sein, sind jedoch zur Steuerung der Absperrventile und der Pumpe nicht erforderlich.

Im folgenden wird unter Bezugnahme auf die Figuren ein Ausführungsbeispiel der Erfindung näher erläutert.

Es zeigen :

Fig. 1 ein Schema der Dialysevorrichtung während der Einlaufphase und

Fig. 2 dieselbe Darstellung wie Fig. 1 in der Rücklaufphase.

Bei dem dargestellten Ausführungsbeispiel ist eine einlumige Nadelkanüle 10, deren Spitze in ein Blutgefäß des Patienten eingestochen wird, mit einer Blutleitung 11 verbunden. Die Blutleitung 11 verzweigt sich in eine Arterienleitung A und eine Venenleitung V. In der Arterienleitung ist ein erstes Absperrventil V1 und in der Venenleitung V ein zweites Absperrventil V2 vorgesehen. Sämtliche hier erwähnten Absperrventile sind ausschließlich Schlauchventile, die die betreffende Schlauchleitung von außen zusammendrücken und den Durchfluß durch diese Schlauchleitung unterbrechen. Die Absperrventile können beispielsweise über Elektromagnete durch ein Steuergerät 16 ferngesteuert werden.

Die Arterienleitung ist hinter dem Absperrventil V1 an den Einlaß der Pumpe 12 angeschlossen, bei der es sich z. B. um eine Schlauchpumpe handelt, deren Drehzahl veränderbar ist. Der Pumpenauslaß ist über das dritte Absperrventil V3 mit dem Einlaß des Dialysators 13 verbunden und der Auslaß des Dialysators 13 ist an den Blasenfänger 14 angeschlossen, dessen Auslaß mit der das zweite Absperrventil V2 enthaltenden Venenleitung V verbunden ist.

Die Expansionskammer 15 befindet sich in einem Parallelweg zur Pumpe 12. Der Einlaß ist über das vierte Absperrventil V4 mit dem Pumpenauslaß verbunden und der Auslaß der Expansionskammer 15 ist über das fünfte Absperrventil V5 mit dem Pumpeneinlaß verbunden.

Der gesamte bisher beschriebene Blutkreislauf einschließlich des Parallelweges zur Pumpe 12 ist als geschlossenes System ausgebildet. Dies bedeutet, daß keine externe Luftzufuhr möglich ist, und daß auch kein Blut aus dem Kreislauf austreten kann. Die Expansionskammer 15 und der Blasenfänger 14 sind dicht geschlossene Behälter.

Sämtliche Absperrventile V1 bis V5 und die Pumpe 12 werden von dem elektronischen Steuergerät 16 in noch zu erläuternder Weise gesteuert.

Die Dialysevorrichtung enthält außerdem einige Druckmeßgeräte zur Überwachung und ggf. Alarmauslösung. Ein erstes Druckmeßgerät D1 ist an die Arterienleitung A angeschlossen, um den Arteriendruck zu messen. Ein zweites Druckmeßgerät D2 mißt den Druck im oberen Bereich des Blasenfängers 14, also den venösen Rücklaufdruck, und ein drittes Druckmeßgerät D3 mißt den Druck im oberen Bereich der Expansionskammer. An dem Gefäß 14 ist noch ein Luftdetektor 17 vorgesehen, der z. B. auf akustischem Wege das Vorhandensein von Luftblasen in dem Blut, das sich im Gefäß 14 befindet, entdeckt. Die Druckmeßgeräte D1 bis D3 und der Luftdetektor 17 können ebenfalls mit dem Steuergerät 16 verbunden sein, um im Falle eines unzulässigen Betriebszustandes Alarm zu geben und/oder notwendige Hilfsmaßnahmen einzuleiten.

In den Zeichnungen sind jeweils die geöffneten Ventile leer und die geschlossenen Ventile voll dargestellt.

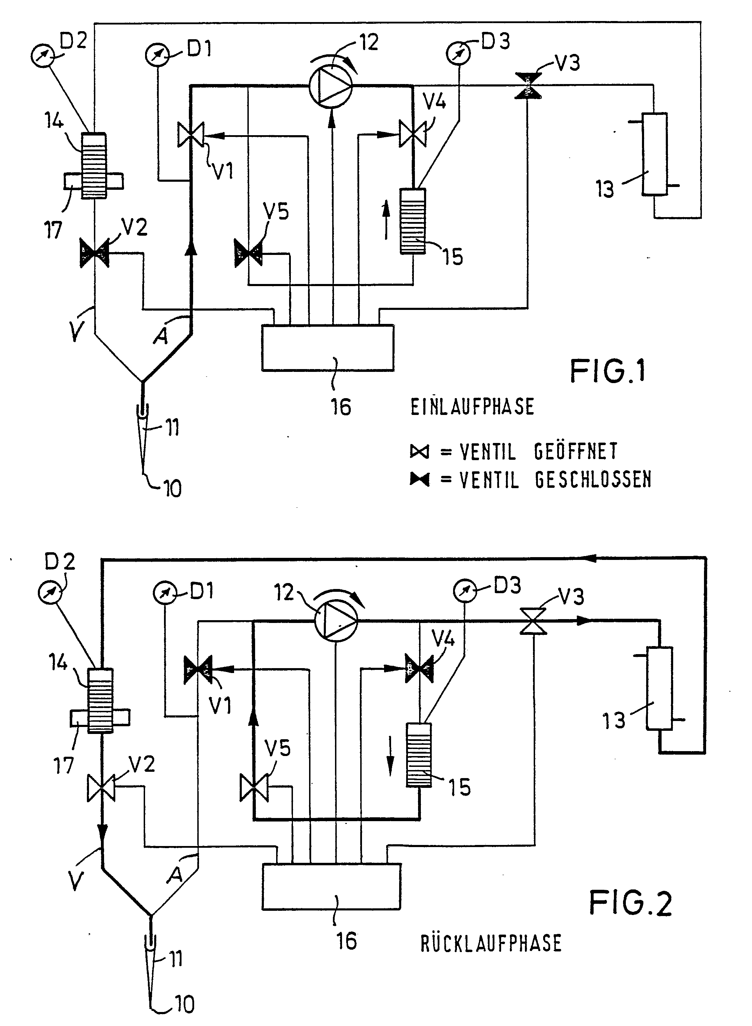

In der Bluteinlaufphase (Fig. 1) läuft zunächst die Blutpumpe 12 mit der maximalen Drehzahl. Sie fördert dabei Blut über die geöffneten Ventile V1 und V4 in die Expansionskammer 15. Hierbei sind die Ventile V2, V3 und V5 geschlossen. Nachdem die Pumpe eine Blutmenge gefördert hat, die dem Füllvolumen der Expansionskammer 15 entspricht, schließt das Steuergerät 16 die Ventile V1 und V4 und es öffnet die Ventile V2, V3 und V5. Die Blutpumpe 12 läuft mit gleicher Drehzahl wie in der Einlaufphase oder mit erhöhter Drehzahl weiter. In der Regel kann die Drehzahl der Pumpe soweit erhöht werden, wie der Patient schmerzfrei durch das zurückströmende Blut bleibt. Das Blut wird nun aus der Expansionskammer 15 durch das geöffnete Ventil V5, die Pumpe 12, das geöffnete Ventil V3, den Dialysator 13, den Blasenfänger 14 und das Ventil V2 zum Patienten zurückgeführt. Diese Phase dauert solange, bis das gesamte Fördervolumen in der Rücklaufphase dem Blutinhalt der Expansionskammer entspricht bzw. ein unterer Pegel in der Expansionskammer erreicht ist. Nach Ablauf dieser Zeitspanne wird die nächste Bluteinlaufphase gestartet.

Die Umschaltung der einzelnen Zyklen erfolgt also zeitgesteuert in Abhängigkeit von der Pumpendrehzahl. Soweit Druckmessungen durchgeführt werden, dienen diese lediglich zur Kontrolle.

Beim Start der Dialysevorrichtung läuft zunächst die Blutpumpe 12 mit den Ventilstellungen gemäß der Einlaufphase (Fig. 1) solange, bis die Expansionskammer 15 gefüllt ist. Hierbei wird das Integral über die Fördermenge der Pumpe 12 gebildet und mit einem dem Füllvolumen der Expansionskammer 15 entsprechenden Wert verglichen. Wenn beide Werte einander gleich geworden sind, schaltet das Steuergerät 16 die Ventilstellungen auf die Rücklaufphase gemäß Fig. 2 um. Hierbei saugt die Pumpe 12 Blut aus der Expansionskammer ab, um dieses Blut über das geöffnete Ventil V3 dem Dialysator 13 zuzuführen. Auch hierbei wird das Integral über die jeweilige Förderleistung der Pumpe gebildet. Es wird also die seit dem Beginn der Rücklaufphase geförderte Blutmenge gemessen und mit dem vorwählbaren Füllvolumen der Expansionskammer 15 verglichen. Wenn der untere Pegelstand erreicht ist, wird der nächste Zyklus ausgeführt.

Während des Betriebes der Dialysevorrichtung kann die Pumpendrehzahl verstellt werden. In diesem Fall ändert sich selbsttätig die Zeit für die Einlaufphase bzw. die Rücklaufphase.

Es ist möglich, daß während der Dialyse die vorgegebenen Druckgrenzen durch fehlerhaften Betrieb oder durch externe Eingriffe unterschritten werden. Aus Sicherheitsgründen erfolgt in einem solchen Fall eine sofortige Umschaltung auf den nächstfolgenden Zyklus. Erst wenn in einer festgelegten Zeitspanne die Druckgrenzen nicht wieder unterschritten werden, wird ein optischer und akustischer Alarm ausgelöst und das System in die sichere Stellung geschaltet.

**Patentansprüche**

1. Dialysevorrichtung zum Dialysieren des Blutes eines Patienten, mit einer einzigen einlumigen Kanüle (10) zum Punktieren eines Blutgefäßes, einer von der Kanüle abgehenden Blutleitung (11), die sich in eine Arterienleitung (A) und eine Venenleitung (V) verzweigt, zwei abwechselnd betätigten Absperrventilen (V1, V2) in der Arterienleitung (A) und der Venenleitung (V), einer über das erste Absperrventil (V1) an die Arterienleitung (A) angeschlossenen einzigen Pumpe (12), einem zwischen Pumpe (12) und Venenleitung (V) geschalteten Dialysator (13) und mit einer Expansionskammer (15) zur vorübergehenden Aufnahme von Blut, dadurch gekennzeichnet, daß die Expansionskammer (15) über je ein Absperrventil (V4, V5) mit dem Auslaß und mit dem Einlaß der Pumpe (12) verbunden ist.

2. Dialysevorrichtung nach Anspruch 1, dadurch gekennzeichnet, daß zwischen den Auslaß der Pumpe (12) und den Dialysator (13) ein weiteres Absperrventil (V3) geschaltet ist, das gleichzeitig mit dem in der Venenleitung (V) liegenden zweiten Absperrventil (V2) gesperrt ist.

3. Dialysevorrichtung nach Anspruch 1 oder 2, dadurch gekennzeichnet, daß das zwischen dem Auslaß der Pumpe (12) und der Expansionskammer (15) liegende vierte Absperrventil (V4) und das zwischen der Expansionskammer (15) und dem Einlaß der Pumpe (12) liegende fünfte Absperrventil (V5) abwechselnd gesperrt sind, wobei das vierte Absperrventil (V4) synchron mit dem in der Arterienleitung (A) liegenden ersten Absperrventil (V1) gesteuert ist.

4. Dialysevorrichtung nach Anspruch 3, dadurch gekennzeichnet, daß das erste und das vierte Absperrventil (V1 und V4) als Doppelventil ausgebildet sind.

5. Dialysevorrichtung nach Anspruch 3 oder 4, dadurch gekennzeichnet, daß das dritte und das fünfte Absperrventil (V3 und V5) als Doppelventil ausgebildet sind.

6. Dialysevorrichtung nach einem der Ansprüche 1 bis 5, dadurch gekennzeichnet, daß zwischen dem Dialysator (13) und der Venenleitung (V) eine Druckmeßvorrichtung (D2) geschaltet ist.

7. Dialysevorrichtung nach einem der Ansprüche 1 bis 6, dadurch gekennzeichnet, daß ein Steuergerät (16) in einer Einlaufphase das Zeitintegral über die Fördermenge der Pumpe (12) bildet und mit einem vorbestimmten Auffüllvolumen der Expansionskammer (15) vergleicht, daß bei Signalgleichheit die Absperrventile (V1 bis V5) auf die Rücklaufphase (Fig. 2) umgeschaltet werden, und daß das Steuergerät (16) auch während der Rücklaufphase das Zeitintegral über die Fördermenge der Pumpe (12) bildet, mit dem Auffüllvolumen vergleicht und bei Signalgleichheit die Absperrventile (V1 bis V5) wieder auf die Einlaufphase (Fig. 1) umschaltet.

**Claims**

1. Dialyzing apparatus for dialyzing the blood of a patient, comprising a single one-bore needle (10) adapted for punctuating a blood vessel, a blood conduit (11) originating from the needle and dividing into an artery conduit (A) and a vein conduit (V), two alternately actuated shut-off valves (V1, V2) in the artery conduit (A) and the vein conduit (V), a single pump (12) connected to the artery conduit (A) via the first shut-off valve (V1), a dialyzer (13) connected between the pump (12) and the vein conduit (V), and an expansion chamber (15) for temporarily receiving blood, characterized in that the expansion chamber (15) is connected to the outlet and to the inlet of the pump (12) via one shut-off valve (V4, V5), respectively.

2. Dialyzing apparatus according to claim 1, characterized in that a further shut-off valve (V3) is connected between the outlet of the pump (12) and the dialyzer (13), which further shut-off valve is closed simultaneously with the second shut-off valve (V2) arranged in the vein conduit (V).

3. Dialyzing apparatus according to claim 1 or 2, characterized in that the fourth shut-off valve (V4) arranged between the outlet of the pump (12) and the expansion chamber (15) and the fifth shut-off valve (V5) arranged between the expansion chamber (15) and the inlet of the pump (12) are alternately closed, the fourth shut-off valve (V4) being controlled synchronously with the first shut-off valve (V1) arranged within the artery conduit (A).

4. Dialyzing apparatus according to claim 3, characterized in that the first and the fourth shut-off valves (V1 and V4) are provided as two-way valves.

5. Dialyzing apparatus according to claim 3 or 4, characterized in that the third and the fifth shut-off valves (V3 and V5) are provided as two-way valves.

6. Dialyzing apparatus according to any one of claims 1 to 5, characterized in that a pressure gauge (D2) is connected between the dialyzer (13) and the vein conduit (V).

7. Dialyzing apparatus according to any one of claims 1 to 6, characterized in that during an intake phase a control device (16) calculates the time integral of the conveying capacity of the pump (12) and compares this time integral to a predetermined filling volume of the expansion chamber (15), that upon equivalence of the signals the shut-off valves (V1 to V5) are switched to the return phase (Fig. 2), and that also during the return phase the control device (16) calculates the time integral of the conveying capacity of the pump (12), compares this time integral to the filling volume, and upon equivalence of the signals switches the shut-off valves (V1 to V5) to the intake phase (Fig. 1) again.

**Revendications**

1. Dispositif de dialyse servant à réaliser la dialyse du sang d'un patient et comportant une canule unique (10) à un orifice servant à perforer un vaisseau sanguin, un conduit (11) de circulation du sang, qui part de la canule et se ramifie en une canalisation (A) pour le sang artériel et une canalisation (V) pour le sang veineux, deux soupapes d'arrivée (V1, V2) actionnées en alternance et situées dans la canalisation (A) pour le sang artériel et dans la canalisation (V) pour le sang veineux, une pompe unique (12) raccordée par l'intermédiaire de la première soupape d'arrêt (V1) à la canalisation (A) pour le sang artériel, un dialyseur (13) branché entre la pompe (12) et la canalisation (V) pour le sang veineux, et une chambre d'expansion (15) servant à recevoir le sang de façon transitoire, caractérisé en ce que la chambre d'expansion (15) est reliée par l'intermédiaire de soupapes d'arrêt respectives (V4, V5) à la sortie et à l'entrée de la pompe (12).

2. Dispositif de dialyse selon la revendication 1, caractérisé en ce qu'entre la sortie de la pompe (12) et le dialyseur (13) se trouve branchée une autre soupape d'arrêt (V3) qui est fermée en même temps que la seconde soupape d'arrêt (V2) située dans la canalisation (V) pour le sang veineux.

3. Dispositif de dialyse selon la revendication 1 ou 2, caractérisé en ce que la quatrième soupape d'arrêt (V4) située entre la sortie de la pompe (12) et la chambre d'expansion (15), et la cinquième soupape d'arrêt (V5) située entre la chambre d'expansion (15) et l'entrée de la pompe (12) sont fermées en alternance, la quatrième soupape d'arrêt (V4) étant commandée de façon synchrone avec la première soupape d'arrêt (V1) située dans la canalisation (A) pour le sang artériel.

4. Dispositif de dialyse selon la revendication 3, caractérisé en ce que les première et quatrième soupapes d'arrêt (V1 et V4) sont réalisées sous la forme de soupapes doubles.

5. Dispositif de dialyse selon la revendication 3 ou 4, caractérisé en ce que les troisième et cinquième soupapes d'arrêt (V3 et V5) sont réalisées sous la forme de soupapes doubles.

6. Dispositif de dialyse selon l'une des revendications 1 à 5, caractérisé en ce qu'un dispositif (D2) de mesure de la pression est branché entre le dialyseur (13) et la canalisation (V) pour le sang veineux.

7. Dispositif de dialyse selon l'une des revendications 1 à 6, caractérisé en ce qu'un appareil de commande (16) forme, pendant la phase aller, l'intégrale de temps du débit de la pompe (12) et compare cette intégrale à un volume de remplissage prédéterminé de la chambre d'expansion (15), que, en cas d'égalité des signaux, les soupapes d'arrêt (V1 à V5) sont commutées sur la phase retour (figure 2) et en ce que l'appareil de commande (16) forme, également pendant la phase retour, l'intégrale de temps du débit de la pompe (12), compare cette intégrale au volume de remplissage et, en cas d'égalité des signaux, commute à nouveau les soupapes d'arrêt (V1 à V5) sur la phase aller (figure 1).

FIG.1

EINLAUFPHASE

⋈ = VENTIL GEÖFFNET
◄► = VENTIL GESCHLOSSEN

FIG.2

RÜCKLAUFPHASE